(19) Europäisches Patentamt
European Patent Office
Office européen des brevets

(11) Veröffentlichungsnummer: **0 034 703**
**B1**

(12) # EUROPÄISCHE PATENTSCHRIFT

(45) Veröffentlichungstag der Patentschrift: **28.09.83**

(51) Int. Cl.³: **C 07 C 103/12,** C 07 C 102/08 // C07D253/06

(21) Anmeldenummer: **81100406.8**

(22) Anmeldetag: **21.01.81**

(54) **Verfahren zur Herstellung von Trimethylbrenztraubensäureamid.**

(30) Priorität: **31.01.80 DE 3003542**

(43) Veröffentlichungstag der Anmeldung: **02.09.81 Patentblatt 81/35**

(45) Bekanntmachung des Hinweises auf die Patenterteilung: **28.09.83 Patentblatt 83/39**

(84) Benannte Vertragsstaaten: **BE CH DE FR GB IT LI NL**

(56) Entgegenhaltungen:
**DE-A-2 708 184**
**DE-A-2 733 181**
**US-A-2 459 686**

(73) Patentinhaber: **BAYER AG, Zentralbereich Patente, Marken und Lizenzen, D-5090 Leverkusen 1, Bayerwerk (DE)**

(72) Erfinder: **Schmidt, Thomas, Dr., Am Bandenfeld 72, D-5657 Haan 1 (DE)**
Erfinder: **Sehnem, Hans-Peter, Nüllerstrasse 90, D-5600 Wuppertal (DE)**

**EP 0 034 703 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Trimethylbrenztraubensäureamid

Die vorliegende Anmeldung betrifft ein Verfahren zur Herstellung des neuen Trimethylbrenztraubensäureamids, welches als Zwischenprodukt zur Synthese von bekannten herbiziden Wirkstoffen verwendet werden kann.

Es ist literaturbekannt, daß die Herstellung von $\alpha$-Ketocarbonsäuren bzw. deren Amiden durch Verseifung von entsprechenden Acylcyaniden grundsätzlich mit erheblichen Schwierigkeiten verbunden ist, da es praktisch nicht gelingt, eine Spaltung der Acylcyanide in Blausäure und 1 C-Atom weniger als das eingesetzte Acylcyanid enthaltende Carbonsäuren ganz zu unterdrücken (Angew. Chem. 68, S. 430 (1956)).

Es ist jedoch bereits bekannt, daß man Brenztraubensäureamid in guter Ausbeute (bis 79%) durch partielle Hydrolyse von Brenztraubensäurenitril erhalten kann, indem man das Brenztraubensäurenitril in etherischer Lösung bei etwa 0°C zunächst mit trocknenem gasförmigem Chlorwasserstoff und anschließend mit Wasser behandelt und das Amid in üblicher Weise isoliert (vgl. J. Amer. Chem. Soc. 73, S. 5914 (1951)).

Weiterhin ist bekanntgeworden, daß man in entsprechender Weise bestimmte C-substituierte Brenztraubensäureamide, nämlich Amide der allgemeinen Formel (I)

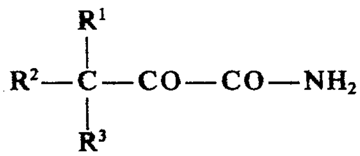 (I)

worin

$R^1$ für Wasserstoff steht;

$R^2$ und $R^3$ gleich oder verschieden sind und für gegebenenfalls durch Halogen substituiertes Alkyl stehen und $R^2$ außerdem für Wasserstoff steht; oder $R^2$ und $R^3$ gemeinsam für einen 3- bis 8gliedrigen Cycloalkylring stehen, der gegebenenfalls durch Alkyl oder Halogen substituiert ist, wobei in diesem Fall $R^1$ auch für Alkyl stehen kann;

in hohen Ausbeuten (bis 91%) erhalten kann, wenn man entsprechende Acylcyanide der allgemeinen Formel (II)

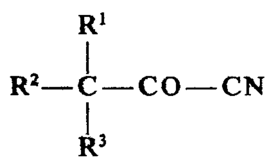 (II)

in welcher $R^1$, $R^2$ und $R^3$ die oben angegebene Bedeutung haben, in Gegenwart eines inerten organischen Lösungsmittels, wie insbesondere Ether, zuerst mit gasförmigem Chlorwasserstoff und dann mit Wasser bei Temperaturen zwischen −70 bis +70°C, vorzugsweise zwischen −40 und +20°C, umsetzt und die Endprodukte in an sich bekannter Weise isoliert (vgl. DE-OS 2 708 184).

Nach den aus dem Stand der Technik bekannten Verfahren ist es dagegen, wie eigene Versuche gezeigt haben, nicht möglich, in entsprechender Weise aus Pivaloylcyanid (Formel II mit $R^1 = R^2 = R^3 =$ Methyl bzw. Formel IV) das — bisher nicht bekannte — Trimethylbrenztraubensäureamid (III) in nennenswerter Ausbeute herzustellen.

Es wurde nun gefunden, daß man das neue Trimethylbrenztraubensäureamid der Formel (III)

$$(CH_3)_3C-CO-CO-NH_2 \qquad (III)$$

in hoher Ausbeute erhält, wenn man Pivaloylcyanid der Formel (IV)

$$(CH_3)_3C-CO-CN \qquad (IV)$$

gegebenenfalls in Gegenwart einer unter den Reaktionsbedingungen flüssigen, niederaliphatischen Carbonsäure als Lösungsmittel und gegebenenfalls unter Druck zuerst mit einer starken, wasserfreien anorganischen Säure und gegebenenfalls danach mit Wasser jeweils bei Temperaturen zwischen −50 und +50°C umsetzt.

Es ist im Hinblick auf den Stand der Technik als ausgesprochen überraschend zu bezeichnen, daß es unter den Bedingungen des erfindungsgemäßen Verfahrens gelingt, Trimethylbrenztraubensäureamid in guter Ausbeute zu erhalten. Insbesondere war aufgrund des Standes der Technik nicht zu erwarten,

daß das erfindungsgemäße Verfahren entweder ohne Lösungsmittel oder in Gegenwart einer niederaliphatischen Carbonsäure als Lösungsmittel in guten Ausbeuten verläuft, zumal diese organischen Säuren unter den Reaktionsbedingungen, insbesondere gegenüber Halogenwasserstoffen, nicht als inert anzusehen sind.

Verwendet man Pivaloylcyanid und Wasser als Ausgangsstoffe, sowie Eisessig als Lösungsmittel und Chlorwasserstoff als anorganische Säure, so kann der Reaktionsablauf durch das folgende Formelschema wiedergegeben werden:

$$(CH_3)_3C-CO-CN \xrightarrow[2.\ H_2O]{1.\ HCl/CH_3COOH} (CH_3)_3C-CO-CO-NH_2$$

Das als Ausgangsstoff zu verwendende Pivaloylcyanid ist bekannt und kann z. B. durch Umsetzung von Pivaloylchlorid mit Kupfer-(I)-cyanid hergestellt werden (vgl. z. B. J. Amer. Chem. Soc. 72, S. 2793 (1950)).

Die Reaktionstemperaturen können bei der Durchführung des erfindungsgemäßen Verfahrens in einem größeren Bereich variiert werden. Im allgemeinen arbeitet man, wie oben angegeben, zwischen −50 und +50°C, vorzugsweise bei −20 bis +30°C.

Die erfindungsgemäße Umsetzung kann unter Normaldruck, aber auch unter erhöhtem Druck, vorzugsweise im Druckbereich von 1 bis 10 bar, durchgeführt werden.

Die erfindungsgemäße Umsetzung wird mit Hilfe einer starken anorganischen Säure durchgeführt. Hierzu gehören vorzugsweise die Halogenwasserstoffsäuren, wie wasserfreier Chlor- und Bromwasserstoff, sowie konzentrierte Schwefelsäure.

Die erfindungsgemäße Umsetzung kann in Abwesenheit oder in Gegenwart einer unter den Reaktionsbedingungen flüssigen, niederaliphatischen Carbonsäure als Lösungsmittel durchgeführt werden. Als solche Lösungsmittel kommen insbesondere Carbonsäuren mit 1 bis 6 C-Atomen, z. B. wasserfreie Essigsäure, Propionsäure oder Ameisensäure in Frage. Besonders bevorzugt ist Eisessig.

Ein Lösungsmittel wird vorzugsweise dann verwendet, wenn man als anorganische Säure einen Halogenwasserstoff einsetzt, während man bei Verwendung einer anorganischen Sauerstoffsäure, wie insbesondere Schwefelsäure, bevorzugt ohne Lösungsmittel arbeitet.

Bei Verwendung einer anorganischen Sauerstoffsäure kann es außerdem zweckmäßig sein, dem Reaktionsgemisch katalytische Mengen an Chloridionen in Form von Alkali- oder Ammoniumchlorid, vorzugsweise 0,01 bis 0,1 Mol pro Mol Sauerstoffsäure, zuzusetzen. Besonders geeignet ist, z. B. bei Verwendung von Schwefelsäure, ein Zusatz an Ammoniumchlorid.

Ein Zusatz von Wasser zum Reaktionsgemisch zur Bildung des Amids (III) ist im Falle, daß eine Carbonsäure als Lösungsmittel und ein Halogenwasserstoff als anorganische Säure verwendet werden, nicht zwingend erforderlich: durch Einwirkung des Halogenwasserstoffs auf die Carbonsäure bildet sich neben einer gewissen Menge Carbonsäurehalogenid die entsprechende Menge Wasser (wobei die Menge von den tatsächlich verwendeten Reagenzien und den tatsächlichen Reaktionsbedingungen abhängt), welche zur quantitativen Hydrolyse des eingesetzten Pivaloylcyanids bzw. möglicher intermediärer Zwischenstufen ausreichend sein kann. — In solchen Fällen fungiert die eingesetzte Carbonsäure nicht nur als Lösungsmittel, sondern zum Teil auch als Reaktionspartner (wobei allerdings auch noch andere Reaktionsmechanismen denkbar sind). Jedenfalls ist es bei dieser Ausführungsform der Erfindung nicht erforderlich, stöchiometrische Mengen an Wasser einzusetzen.

Bei der Durchführung des erfindungsgemäßen Verfahrens setzt man auf 1 Mol Pivaloylcyanid (IV) im allgemeinen 1 bis 10 Mol an anorganischer Säure und 0 bis 10 Mol Wasser, vorzugsweise 1 bis 8 Mol an anorganischer Säure und 0 bis 2,5 Mol Wasser ein.

Zur Isolierung des Trimethylbrenztraubensäureamids (III) wird das Reaktionsgemisch neutralisiert und in üblicher Weise aufgearbeitet.

Das Trimethylbrenztraubensäureamid kann in an sich bekannter Weise, durch saure oder alkalische Hydrolyse, zur freien Trimethylbrenztraubensäure (3,3-Dimethyl-2-oxo-buttersäure) verseift werden. Damit ermöglicht das erfindungsgemäße Verfahren erstmals auch die direkte Hydrolyse von Pivaloylcyanid zu Trimethylbrenztraubensäure, wobei das Amid-Zwischenprodukt nicht isoliert zu werden braucht.

Dies bedeutet gegenüber dem aus dem Stand der Technik bekannten Verfahren zur Herstellung von Trimethylbrenztraubensäure aus Pivaloylcyanid (vgl. DE-AS 2 733 181) eine vorteilhafte Verfahrensvereinfachung, da der »Umweg« über die Ritter-Reaktion und damit über das N-t-Butylamid der Trimethylbrenztraubensäure vermieden wird. Das erfindungsgemäße Verfahren verläuft ohne den Hilfsstoff t-Butanol bzw. Isobutylen, der bei der vorbekannten Ritter-Reaktion als zusätzliches Reagenz erforderlich ist.

Trimethylbrenztraubensäureamid (III) ist damit — ebenso wie die freie Trimethylbrenztraubensäure — ein wertvolles Zwischenprodukt zur Herstellung von bestimmten herbiziden Wirkstoffen aus der Reihe der asymmetrischen Triazinone, z. B. von 4-Amino-6-tert.-butyl-3-methylthio-1,2,4-triazin-5(4H)-on (VII) (vgl. DE-PS 1 795 784, DE-AS 2 648 300, DE-OS 2 460 889, DE-OS 2 165 554).

Trimethylbrenztraubensäureamid (III) kann entweder direkt (vgl. nachfolgendes Formelschema und Beispiel B) oder nach vorheriger Verseifung zur freien Ketosäure in halogenwasserstoffsaurer,

wäßriger Lösung mit 1 bis 1,5 Mol Thiocarbohydrazid (V) bei Temperaturen zwischen 20° und 100° C zu 4-Amino-6-tert.-butyl-3-mercapto-1,2,4-triazin-5(4H)-on (VI) kondensiert werden, welches sich in bekannter Weise mittels Methylhalogenid, z. B. Methyljodid oder Methylbromid, in alkalischer Lösung zu 4-Amino-6-tert.-butyl-3-methylthio-1,2,4-triazin-5(4H)-on (VII) methylieren läßt:

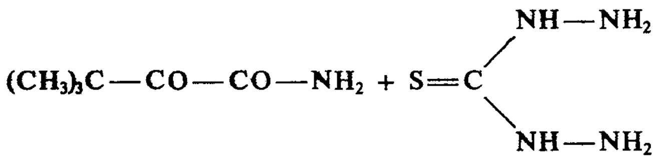

(III) (V)

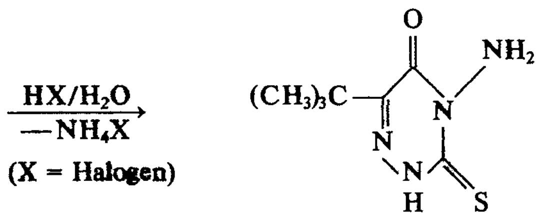


(VI)

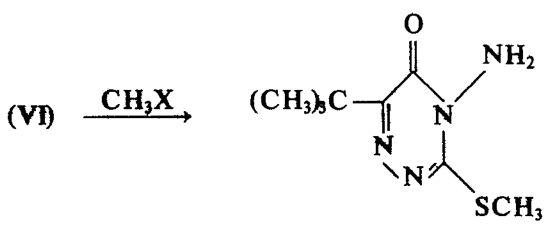


(VII)

Das Trimethylbrenztraubensäureamid (III) hat dabei gegenüber dem vorbekannten Trimethylbrenztraubensäure-N-t-butylamid den Vorteil, daß es sich wie oben erläutert, einfacher herstellen läßt und daß bei der Weiterverarbeitung — sei es durch Hydrolyse oder durch direkte Kondensation mit Thiocarbohydrazid — als Nebenprodukt lediglich Ammoniak in Form von Ammoniumsalzen anfällt.

Die nachfolgenden Herstellungsbeispiele sollen das erfindungsgemäße Verfahren näher erläutern.

## Herstellungsbeispiele

### A) Herstellung von Trimethylbrenztraubensäureamid

$(CH_3)_3C-CO-CO-NH_2$

### Beispiel 1

Zu 800 g einer 36%igen Lösung von Bromwasserstoff in Eisessig werden bei Raumtemperatur unter Rühren 111,0 g (1 Mol) Pivaloylcyanid getropft. Nach beendeter Zugabe läßt man drei Stunden bei Raumtemperatur nachrühren. Danach tropft man bei 20 bis 25° C 9 ml (0,5 Mol) Wasser hinzu und rührt 1 Stunde bei Raumtemperatur nach. Anschließend gießt man die Reaktionslösung in einen Überschuß gesättigter Natriumhydrogencarbonatlösung. Man extrahiert dreimal mit je 200 ml Methylenchlorid. Die vereinigten organischen Phasen werden mit 200 ml Wasser gewaschen, über Natriumsulfat getrocknet und durch Abdestillieren des Lösungsmittels im Vakuum eingeengt. Das zurückbleibende Öl kristallisiert nach einiger Zeit durch. Nach Umkristallisation aus Petrolether erhält man 113,5 g (88% der Theorie) Trimethylbrenztraubensäureamid vom Schmelzpunkt 69 – 70° C.

### Beispiel 2

In eine Mischung aus 60 g (1 Mol) Eisessig und 111,0 g (1 Mol) Pivaloylcyanid wird 5 Stunden bei 20 – 25° C/8 bar Chlorwasserstoff eingeleitet. Anschließend wird entspannt und das Reaktionsgemisch auf einen Überschuß einer gesättigten Natriumhydrogencarbonatlösung gegossen. Man extrahiert dreimal mit je 200 ml Methylenchlorid. Die vereinigten organischen Phasen werden mit 200 ml Wasser gewaschen, über Natriumsulfat getrocknet und durch Abdestillieren des Lösungsmittels im Vakuum

eingeengt. Der zurückbleibende Rückstand kristallisiert nach kurzer Zeit. Nach Umkristallisation aus Petrolether erhält man 97 g (75,2% der Theorie) Trimethylbrenztraubensäureamid vom Schmelzpunkt 68–69°C.

Beispiel 3

In einen 500 ml Dreihalskolben gibt man 55,5 g (0,5 Mol) Pivaloylcyanid und 2 g Ammoniumchlorid. Man kühlt auf –5 bis 0°C ab und tropft bei dieser Temperatur 100 ml konzentrierte Schwefelsäure zu. Danach tropft man ebenfalls bei –5 bis 0°C 18 ml (1 Mol) Wasser zu. Man läßt 12 Stunden bei –5 bis 0°C nachrühren und gießt dann das Reaktionsgemisch auf eine Lösung von 350 g Natriumcarbonat in 600 ml Wasser. Man extrahiert zweimal mit je 300 ml Methylenchlorid. Die vereinigten organischen Phasen werden mit 300 ml Wasser gewaschen, über Natriumsulfat getrocknet und durch Abdestillieren des Lösungsmittels im Vakuum eingeengt. Nach Umkristallisation aus Petrolether erhält man 66 g (51,2% der Theorie) Trimethylbrenztraubensäureamid vom Schmelzpunkt 67–69°C.

B) Herstellung von 4-Amino-6-tert.-butyl-3-methylthio-1,2,4-triazin-5(4H)-on (VII)

1. und 2. Stufe

Zu 800 g einer Lösung von Bromwasserstoff in Eisessig (36%ig) werden 111,0 g (1 Mol) Pivaloylcyanid bei Raumtemperatur zugetropft. Nach beendeter Zugabe rührt man drei Stunden bei Raumtemperatur nach. Anschließend tropft man 9 ml (0,5 Mol) Wasser bei 20 bis 25°C zu und läßt eine Stunde bei Raumtemperatur nachrühren. Danach werden der Eisessig und der überschüssige Bromwasserstoff im Vakuum abgezogen und das anfallende Trimethylbrenztraubensäureamid wird in eine Mischung von 107,6 g Thiocarbohydrazid, 420 g Wasser und 140,6 g konzentrierte Salzsäure eingebracht. Diese Reaktionsmischung rührt man 1,5 Stunden bei 90°C und 12 Stunden bei Raumtemperatur nach. Danach wird auf 0°C abgekühlt und das ausfallende Kristallisat abfiltriert, mit 200 ml Wasser gewaschen und bei 100°C im Vakuum getrocknet. Man erhält 168,6 g (84,3% der Theorie) 4-Amino-6-tert.-butyl-3-mercapto-1,2,4-triazin-5(4H)-on vom Schmelzpunkt 205–209°C.

3. Stufe

In eine Mischung von 97 g 45%iger Natronlauge und 65 g Wasser werden unter Rühren 20 g (0,1 Mol) 4-Amino-6-tert.-butyl-3-mercapto-1,2,4-triazin-5(4H)-on eingetragen. Nach vollständiger Lösung des Produktes werden 16,5 g Methyljodid so zugegeben, daß die Innentemperatur 30°C nicht übersteigt. Nach beendeter Zugabe wird die Reaktionslösung noch zwei Stunden bei Raumtemperatur gerührt. Danach wird der entstandene Feststoff abgesaugt, mit 100 ml Wasser gewaschen und im Vakuumtrockenschrank bei 60°C getrocknet. Man erhält 17,3 g (81% der Theorie) 4-Amino-6-tert.-butyl-3-methylthio-1,2,4-triazin-5(4H)-on vom Schmelzpunkt 123–125°C.

**Patentansprüche**

1. Trimethylbrenztraubensäureamid der Formel III

$$(CH_3)_3C-CO-CO-NH_2 \qquad (III).$$

2. Verfahren zur Herstellung von Trimethylbrenztraubensäureamid (III) durch partielle Hydrolyse von Pivaloylcyanid der Formel IV

$$(CH_3)_3C-CO-CN \qquad (IV),$$

dadurch gekennzeichnet, daß man das Pivaloylcyanid gegebenenfalls in Gegenwart einer unter den Reaktionsbedingungen flüssigen, niederaliphatischen Carbonsäure als Lösungsmittel und gegebenenfalls unter Druck zuerst mit einer starken, wasserfreien anorganischen Säure und gegebenenfalls danach mit Wasser jeweils bei Temperaturen zwischen –50 und +50°C umsetzt.

3. Verfahren nach Anspruch 2, dadurch gekennzeichnet, daß man bei Temperaturen zwischen –20 und +30°C arbeitet.

4. Verfahren nach Anspruch 2, dadurch gekennzeichnet, daß man im Druckbereich von 1 bis 10 bar arbeitet.

5. Verfahren nach Anspruch 2, dadurch gekennzeichnet, daß man auf 1 Mol Pivaloylcyanid 1 bis 10 Mol an anorganischer Säure und 0 bis 10 Mol Wasser einsetzt.

6. Verfahren nach Anspruch 2, dadurch gekennzeichnet, daß man auf 1 Mol Pivaloylcyanid 1 bis 8 Mol an anorganischer Säure und 0 bis 2,5 Mol Wasser einsetzt.

7. Verfahren nach Anspruch 2, dadurch gekennzeichnet, daß man als Lösungsmittel eine Carbonsäure mit 1 bis 6 C-Atomen einsetzt.

8. Verfahren nach Anspruch 7, dadurch gekennzeichnet, daß man als Lösungsmittel Eisessig einsetzt.

9. Verfahren nach Anspruch 2, dadurch gekennzeichnet, daß man als Lösungsmittel Eisessig und als anorganische Säure Chlorwasserstoff oder Bromwasserstoff verwendet.

10. Verfahren nach Anspruch 2, dadurch gekennzeichnet, daß man in Abwesenheit eines Lösungsmittels arbeitet und als anorganische Säure konzentrierte Schwefelsäure einsetzt.

## Claims

1. Trimethylpyruvic acid amide ot the formula III

$$(CH_3)_3C-CO-NH_2 \qquad (III).$$

2. Process for the preparation of trimethylpyruvic acid amide (III) by partial hydrolysis of pivaloyl cyanide of the formula IV

$$(CH_3)_3C-CO-CN \qquad (IV),$$

characterised in that pivaloyl cyanide is reacted, if appropriate in the presence of a lower aliphatic carboxylic acid which is liquid under the reaction conditions, as the solvent, and if appropriate under pressure, first with a strong, anhydrous inorganic acid and then, if appropriate, with water, in each case at temperatures between $-50$ and $+50°$ C.

3. Process according to Claim 2, characterised in that the reaction is carried out at temperatures between $-20$ and $+30°$ C.

4. Process according to Claim 2, characterised in that the reaction is carried out in the pressure range from 1 to 10 bars.

5. Process according to Claim 2, characterised in that 1 to 10 mols of inorganic acid and 0 to 10 mols of water are employed per 1 mol of pivaloyl cyanide.

6. Process according to Claim 2, characterised in that 1 to 8 mols of inorganic acid and 0.1 to 2.5 mols of water are employed per 1 mol of pivaloyl cyanide.

7. Process according to Claim 2, characterised in that a carboxylic acid with 1 to 6 carbon atoms is employed as the solvent.

8. Process according to Claim 7, characterised in that glacial acetic acid is employed as the solvent.

9. Process according to Claim 2, characterised in that glacial acetic acid is used as the solvent and hydrogen chloride or hydrogen bromide is used as the inorganic acid.

10. Process according to Claim 2, characterised in that it is carried out in the absence of a solvent and concentrated sulphuric acid is employed as the inorganic acid.

## Revendications

1. L'amide d'acide triméthylpyruvique de formule III

$$(CH_3)_3C-CO-CO-NH_2 \qquad (III).$$

2. Procédé de production d'amide d'acide triméthylpyruvique (III) par hydrolyse partielle de cyanure de pivaloyle de formule IV

$$(CH_3)_3C-CO-CN \qquad (IV)$$

caractérisé en ce qu'on fait réagir le cyanure de pivaloyle, éventuellement en présence d'un acide carboxylique aliphatique inférieur liquide dans les conditions réactionnelles, utilisé comme solvant, et le cas échéant sous pression, tout d'abord avec un acide inorganique fort anhydre, puis, le cas échéant, avec de l'eau, dans chaque cas, à des températures comprises entre $-50$ et $+50°$ C.

3. Procédé suivant la revendication 2, caractérisé en ce qu'on opère à des températures comprises entre $-20$ et $+30°$ C.

4. Procédé suivant la revendication 2, caractérisé en ce qu'on opère dans la plage de pressions de 1 à 10 bars.

5. Procédé suivant la revendication 2, caractérisé en ce qu'on utilise, par mole de cyanure de pivaloyle, 1 à 10 moles d'acide inorganique et 0 à 10 moles d'eau.

6. Procédé suivant la revendication 2, caractérisé en ce qu'on utilise par mole de cyanure de pivaloyle 1 à 8 moles d'acide inorganique et 0 à 2,5 moles d'eau.

7. Procédé suivant la revendication 2, caractérisé en ce qu'on utilise comme solvant un acide carboxylique ayant 1 à 6 atomes de carbone.

8. Procédé suivant la revendication 7, caractérisé en ce qu'on utilise comme solvant l'acide acétique cristallisable.

9. Procédé suivant la revendication 2, caractérisé ence qu'on utilise comme solvant l'acide acétique cristallisable et comme acide inorganique le chlorure d'hydrogène ou le bromure d'hydrogène.

10. Procédé suivant la revendication 2, caractérisé en ce qu'on opère en l'absence d'un solvant et on utilise comme acide inorganique l'acide sulfurique concentré.